(19) Europäisches Patentamt
European Patent Office
Office européen des brevets

(11) **EP 1 783 484 A1**

(12) **EUROPÄISCHE PATENTANMELDUNG**

(43) Veröffentlichungstag:
**09.05.2007 Patentblatt 2007/19**

(51) Int Cl.:
*G01N 27/08* (2006.01)   *G01N 27/49* (2006.01)
*G01N 27/10* (2006.01)   *G01N 33/487* (2006.01)

(21) Anmeldenummer: **05024074.6**

(22) Anmeldetag: **04.11.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder:
• **F.HOFFMANN-LA ROCHE AG**
 **4070 Basel (CH)**
 Benannte Vertragsstaaten:
 **AT BE BG CH CY CZ DK EE ES FI FR GB GR HU IE IS IT LI LT LU LV MC NL PL PT RO SE SI SK TR**
• **Roche Diagnostics GmbH**
 **68305 Mannheim (DE)**
 Benannte Vertragsstaaten:
 **DE**

(72) Erfinder: **Zaugg, Claudio**
 **4900 Langenthal (CH)**

(74) Vertreter: **Blum, Rudolf Emil**
 **E. BLUM & CO. AG**
 **Patent- und Markenanwälte VSP**
 **Vorderberg 11**
 **8044 Zürich (CH)**

(54) **System und Verfahren zur Bestimmung der Konzentration einer Substanz in einer Flüssigkeit**

(57) Ein Analysesystem (1) hat eine Messzelle (2) zur Bestimmung einer Konzentration einer Substanz in einer Flüssigkeit. Die Messzelle (2) hat einen Sensor (14), der ein der Konzentration entsprechendes Signal erzeugt, wenn die Flüssigkeit dem Sensor (14) über einen ersten Kanal (4) zugeführt und über einen zweiten Kanal (6) von ihm abgeführt wird. Eine Einrichtung (8, 10, 12) zur Messung einer Leitfähigkeit der am Sensor (14) vorbeiströmenden Flüssigkeit ist an die Messzelle (2) gekoppelt.

Fig. 1

EP 1 783 484 A1

**Beschreibung**

Technisches Gebiet

Die Erfindung betrifft allgemein das Gebiet der medizinischen Analytik und Diagnostik, insbesondere ein System und ein Verfahren zur Bestimmung einer Konzentration einer Substanz in einer Flüssigkeit.

Stand der Technik

In Bereichen der medizinischen Analytik und Diagnostik werden biochemische in-vivo Prozesse nebst in Blut auch in interstitieller Gewebeflüssigkeit gemessen. Dazu gehört beispielsweise die Messung von Glukose mittels der Mikrodialyse oder der sogenannten "open-flow-microperfusion". Bei der Mikrodialyse wird über einen zuführenden Schlauch eine Perfusionslösung (Perfusat) zu einer semipermeablen Membran gepumpt. Dort kommt es zu einem Stoffaustausch zwischen der interstitiellen Flüssigkeit und einem inneren Lumen der Membran. Über einen abführenden Schlauch wird das beim Stoffaustausch erzeugte Dialysat abgeführt und kann in weiteren analysiert werden. Bei der genannten Mikroperfusion wird über einen subkutan liegenden doppellunigen Katheter eine Lösung mit physiologischer Osmolarität in den interstitiellen Raum gepumpt und zur weiteren Analyse wieder abgesogen.

Die weitere Analyse des Dialysats oder der abgesogenen physiologischen Lösung kann mit einem Biosensor, basierend auf $H_2O_2$ produzierenden oder verbrauchenden Oxidoreductasen, erfolgen. Der Biosensor ist beispielsweise ein in Dickfilmtechnologie hergestelltes Halbleiterelement. Mit Hilfe des Enzyms Glukoseoxidase, das auf einer Elektrode des Biosensors immobilisiert ist, kommt es zu einer katalytischen Reaktion, bei der entstehendes Wasserstoffperoxid als Nachweismolekül dient und an einer Platinelektrode oxidiert wird.

Die bei der oxidation entstehenden Elektronen bilden einen Strom, der proportional der Glukosekonzentration ist. Die Messung erfolgt amperometrisch mit einem Potentiostat, d. h. auf einer Strommessung bei konstantem Potential. Zwischen einer Referenzelektrode und einer Arbeitselektrode liegt ein definiertes Potential, das vom Potentiostat konstant gehalten wird. Bezugspunkt ist dabei eine Referenzelektrode. Die Gegenelektrode dient der Regelung und hat ein schwankendes Potenzial.

Bei den genannten Messverfahren tritt der Effekt auf, dass die gemessenen Konzentrationen der interessierenden Substanzen (z. B. Glucose) je nach Messverfahren von den Werten im analysierten Kompartiment abweichen können. Dies ist auf einem unvollständigen Ausgleich zwischen der Perfusions- und der Gewebeflüssigkeit zurückzuführen. Die absoluten Konzentrationen im Dialysat und in der interstitiellen Flüssigkeit stimmen somit nicht überein. Um dies zu berücksichtigen wird die sogenannte "Recovery" benutzt, die generell ein Mass für das Wiederfinden von interstitiellen Substanzen (z. B. Glukose) im Dialysat ist. Die Recovery wird aus dem Quotienten der Konzentration einer Substanz im Dialysat und der wahren Konzentration der Stammlösung oder der interstitiellen Lösung gebildet und üblicherweise in Erozent angegeben.

Bei den beschrieben Verfahren wird das Dialysat in einem Behälter gesammelt. In einem separaten Analyseverfahren wird für dieses Dialysat dann die Recovery bestimmt. Daran anschliessend wird der amperiometrisch bestimmte Glukosewert durch die Recovery korrigiert. Die Glukosewerte werden daher mit einer Verzögerung bestimmt, so dass die Bestimmung von Glukosewerten mit Berücksichtigung der Recovery in Echtzeit nicht möglich ist.

Darstellung der Erfindung

Es stellt sich daher die Aufgabe, die Bestimmung von in interstitieller Gewebeflüssigkeit vorhandenen Substanzen zu verbessern, so dass eine Messung in Echtzeit möglich ist. Die im Folgenden beschriebene-Technologie ermöglicht die kontinuierliche Messung solcher Substanzen bei parallel dazu erfolgender Bestimmung der Recovery. Ein um die Recovery korrigierter Messwert, beispielsweise ein Glukosewert, steht somit in Echtzeit zur Verfügung.

Ein Aspekt betrifft daher ein Analysesystem, das eine Messzelle zur Bestimmung einer Konzentration einer Substanz in einer Flüssigkeit hat. Die Messzelle hat einen Sensor, der ein der Konzentration entsprechendes Signal erzeugt, wenn die Flüssigkeit dem Sensor über einen ersten Kanal zugeführt und über einen zweiten Kanal von ihm abgeführt wird. Eine Einrichtung zur Messung einer Leitfähigkeit der am Sensor vorbeiströmenden Flüssigkeit ist an die Messzelle gekoppelt.

Ein weiterer Aspekt betrifft ein Verfahren zur Bestimmung einer Konzentration einer Substanz in einer Flüssigkeit. Die Flüssigkeit wird einem Sensor über einen ersten Kanal zugeführt und über einen zweiten Kanal von ihm abgeführt. Dabei erzeugt der Sensor ein der Konzentration entsprechendes elektrisches Signal. Während die Flüssigkeit am Sensor vorbeiströmt wird eine Leitfähigkeit der Flüssigkeit bestimmt.

Die Leitfähigkeit und die Konzentration werden mit Hilfe derselben Messzelle ermittelt. Über die Leitfähigkeit wird die Recovery bestimmt, mittels derer die mit dem Sensor gemessene Konzentration korrigiert wird. Dies erfolgt in Echtzeit.

Kurze Beschreibung der Zeichnung

Weitere Ausgestaltungen, Vorteile, neue Eigenschaften und Anwendungen der Erfindung ergeben sich aus der folgenden detaillierten Beschreibung unter Einbezug der Zeichnung. In der einziger Zeichnung zeigt:

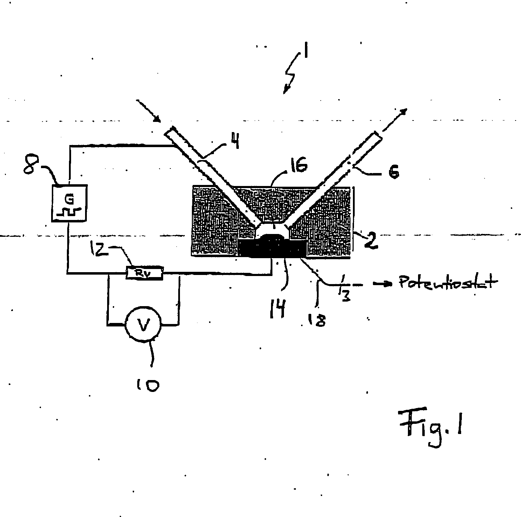

Fig. 1 eine schematische Darstellung eines Messsystems.

Wege zur Ausführung der Erfindung

Die verschiedenen Wege zur Ausführung der Erfindung sind im Folgenden ohne Einschränkung des Schutzbereiches mit Bezug auf die Bestimmung der Konzentration von Glukose in einer Körperflüssigkeit beschrieben. Glukose ist jedoch nur ein Beispiel für eine diagnostisch und therapeutisch wichtige Substanz. Beispiele anderer Substanzen sind Laktat oder Alkohol.

Figur 1 zeigt eine schematische Darstellung eines Ausführungsbeispiels eines Analysesystems 1. Das gezeigte Analysesystem 1 hat eine Messzelle 2, die mit zwei Fluidkanälen 4, 6 verbunden ist, einen Spannungsgenerator 8, einen Messwiderstand ($R_V$) 12 und eine Einrichtung 10 zur Messung einer am Messwiderstand 12 abfallenden Spannung. Im gezeigten Ausführungsbeispiel ist ein Anschluss des Spannungsgenerators 8 mit dem Fluidkanal 4 verbunden und ein weiterer Anschluss mit dem Messwiderstand 12. Der Messwiderstand 12 ist ausserdem mit der Messzelle 2 verbunden.

Die Messzelle 2 hat einen Sensor 14, beispielsweise einen Biosensor, der von einer Messkammer 16 abgedeckt ist. Der Sensor 14 ist in einem Ausführungsbeispiel ein kommerziell erhältlicher Glukosesensor, beispielsweise ein enzymatischer, auf Glukoseoxidase basierender Glukosesensor. Der Sensor 14 hat eine Arbeitselektrode, eine Referenzelektrode und eine Gegenelektrode, die über eine Verbindung 18 mit einem Potentiostat verbunden sind.

Die Fluidkanäle 4, 6 münden in die Messkammer 16. Über den Fluidkanal 4 kann eine zu untersuchende Flüssigkeit in die Messkammer 16 geleitet werden, und über den Fluidkanal 6 kann sie aus der Messkammer 16 wieder abgeleitet werden. Während die Flüssigkeit am Sensor 14 vorbeiströmt, reagiert die Flüssigkeit mit dem auf der Arbeitselektrode immobilisiert vorhandenen Enzym Glukoseoxidase. Diese Reaktion verursacht einen Strom, der zu der Substratkonzentration proportional ist und vom angeschlossenen Potentiostat gemessen wird.

Im in Figur 1 gezeigten Analysesystem 1 kann neben der beschriebenen Glukosemessung zusätzlich die Leitfähigkeit der Dialysatflüssigkeit gemessen werden. Die Leitfähigkeit dient zur Bestimmung der Recovery. Zur Bestimmung der Leitfähigkeit ist der Spannungsgenerator 8, wie bereits beschrieben, mit dem Fluidkanal 4 verbunden, durch den die Dialysatflüssigkeit in die Messkammer 16 strömt. Der Fluidkanal 4 ist aus einem Material, das eine gute elektrische Leitfähigkeit hat und sich chemisch neutral gegenüber der Dialysatflüssigkeit verhält, beispielsweise Edelstahl. Der Widerstand 12 ist indirekt über Masse der Schaltung mit der Arbeits-und/oder der Gegenelektrode des Sensors 14 verbunden.

Im gezeigten Ausführungsbeispiel ist der Spannungsgenerator 8 mit dem Fluidkanal 4 verbunden, Der Fluidkanal 4 dient daher als Zuflusskanal und zusätzlich als Elektrode für die Leitfähigkeitsmessung. In einem anderen Ausführungsbeispiel kann der Spannungsgenerator 8 mit einer separate Elektrode verbunden sein, die in der Messzelle 2 so positioniert ist, dass sie in Kontakt mit der Dialysatflüssigkeit ist. Diese Elektrode kann beispielsweise in die Messkammer 16 reichen.

Die Dialysatflüssigkeit ist ein Elektrolyt mit einer bestimmten Leitfähigkeit. Die zwischen dem Fluidkanal 4 und den beiden Elektroden (Arbeits- und Gegenelektrode) angelegte Spannung verursacht einen Elektronenfluss, der innerhalb der Messkammer 16 den Stromkreis schliesst. Die Stärke des Elektronenflusses, d. h. des Stromes, hängt von der Leitfähigkeit der Dialysatflüssigkeit ab.

Um eine chemische Änderung der Dialysatflüssigkeit, beispielsweise durch Elektrolyse, zu vermeiden, hat die vom Spannungsgenerator 8 erzeugte Spannung keine Gleichstromkomponente sondern ausschliessliche eine Wechselstromkomponente. Die Frequenz der Wechselspannung beträgt in einem Ausführungsbeispiel ca. 1 kHz. Am Messwiderstand 12 fällt daher auch eine Wechselspannung ab, die eine Gleichrichterschaltung in eine Gleichspannung wandelt.

Die Recovery wird gemäss der folgenden Gleichung berechnet:

$$r = r_0 \cdot G/G_0,$$

wobei

    $r$ Recovery,
    $r_0$ Referenzrecovery,
    $G$ Leitfähigkeit [$\mu S$], und
    $G_0$ Referenzleitfähigkeit [$\mu S$].

Die Referenzrecovery $r_0$ einer bekannten Referenzflüssigkeit, welche dieselbe Leitfähigkeit wie die zu analysierende Flüssigkeit hat, ist beispielsweise 1, d. h. 100 %. Diese Flüssigkeit wird zur Bestimmung der Referenzleitfähigkeit $G_0$ während der Kalibrierung des Systems 1 verwendet. Die Referenzflüssigkeit wird mittels der Fluidkanäle 4, 6 am Sensor 14 vorbeigeführt, wobei die Leitfähigkeit der Flüssigkeit bestimmt wird.

Die im vorhergehenden beschriebenen Ausführungsbeispiele eines Analysesystems ermöglichen die kontinuierliche Bestimmung der Konzentration von beispielsweise Glukose bei parallel dazu erfolgender Bestimmung der Recovery. Die Messungen zur Bestimmung der Glukosekonzentration und der Recovery über die Leitfähigkeit erfolgt dabei am selben Crt, d. h. in der Messzelle 2. Eigenschaften des Systems (z. B. Geometrie der Messzelle) wirken sich somit, soweit sie sich auf ein Messsignal auswirken, auf beide Messungen im wesentlichen gleich aus. Luftblasen, beispielsweise, welche in die Messzelle 2 gelangen und möglicherweise einen negativen Einfluss auf die Resultate des Biosensors haben können, werden festgestellt. Hinzukommt, dass das Analysesystem 1 als relativ kleine Einheit mit einer Doppelfunktion realisieren lässt. Das System kommt deshalb auch mit einem sehr kleinen Volumen an Dialysatflüssigkeit aus.

**Patentansprüche**

1. Analysesystem (1) mit einer Messzelle (2) zur Bestimmung einer Konzentration einer Substanz in einer Flüssigkeit, bei dem die Messzelle (2) einen Sensor (14) hat, der ein der Konzentration entsprechendes Signal erzeugt, wenn die Flüssigkeit dem Sensor (14) über einen ersten Kanal (4) zugeführt und über einen zweiten Kanal (6) vom Sensor (14) abgeführt wird, und bei dem eine Einrichtung (8, 10, 12) zur Messung einer Leitfähigkeit der am Sensor (14) vorbeiströmenden Flüssigkeit an die Messzelle (2) gekoppelt ist.

2. Analysesystem (1) nach Anspruch 1, bei dem der erste Kanal (4) aus leitfähigem Material ist, und bei dem die Einrichtung (8, 10, 12) an den ersten Kanal (4) und an eine erste Elektrode des Sensors (14) gekoppelt ist.

3. Analysesystem (1) nach Anspruch 2, bei dem die Einrichtung (8, 10, 12) einen Spannungsgenerator (8), einen Messwiderstand (12) und eine Spannungsmesseinrichtung (10) hat, wobei der Spannungsgenerator (8) an den ersten Kanal (4) und den Messwiderstand (12) gekoppelt ist, und wobei der Messwiderstand (12) die erste Elektrode des Sensors (14) gekoppelt ist.

4. Analysesystem (1) nach Anspruch 1, bei dem die Messzelle (2) eine Elektrode hat, die in Kontakt mit der zugeführten Flüssigkeit steht, und bei dem die Einrichtung (8, 10, 12) einen Spannungsgenerator (8), einen Messwiderstand (12) und eine Spannungsmesseinrichtung (10) hat, wobei der Spannungsgenerator (8) an die Elektrode und den Messwiderstand (12) gekoppelt ist, und wobei der Messwiderstand (12) die erste Elektrode des Sensors (14) gekoppelt ist.

5. Analysesystem (1) nach einem der vorhergehenden Ansprüche, bei dem der Sensor (14) ein Sensor für Glukose oder Laktat ist.

6. Analysesystem (1) nach einem der vorhergehenden Ansprüche, bei dem der Sensor (14) drei Elektroden für eine amperiometrische Messung hat.

7. Verfahren zur Bestimmung einer Konzentration einer Substanz in einer Flüssigkeit,

bei dem die Flüssigkeit einem Sensor (14) über einen ersten Kanal (4) zugeführt und über einen zweiten Kanal (6) von ihm abgeführt wird, wobei der Sensor (14) ein der Konzentration entsprechendes Signal erzeugt; und
bei dem während die Flüssigkeit am Sensor (14) vorbeiströmt eine Leitfähigkeit der Flüssigkeit bestimmt wird.

8. Verfahren nach Anspruch 7, bei dem die Leitfähigkeit zur Bestimmung einer Recovery verwendet wird.

9. Verfahren nach Anspruch 8, bei dem die dem Signal entsprechende Konzentration um die Recovery korrigiert wird.

10. Verfahren nach Anspruch 9, bei dem in einer Kalibrierungsphase eine Referenzleitfähigkeit und eine Referenzrecovery einer Referenzflüssigkeit bestimmt wird.

Fig. 1

**Europäisches Patentamt**

**EUROPÄISCHER RECHERCHENBERICHT**

Nummer der Anmeldung

EP 05 02 4074

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/168934 A1 (SCHAUPP LUKAS ET AL) 2. September 2004 (2004-09-02) * Absätze [0008], [0015], [0025], [0027], [0044], [0055], [0067]; Abbildung 10 * | 1-10 | G01N27/08 G01N27/49 G01N27/10 G01N33/487 |
| X | WO 97/16726 A (CHIRON DIAGNOSTICS CORPORATION; SULLIVAN, KEVIN, J) 9. Mai 1997 (1997-05-09) * Seite 2, Zeile 5 - Zeile 19; Abbildung 6 * * Seite 4, Zeilen 20,21 * | 1 | |
| A | US 6 852 500 B1 (HOSS UDO ET AL) 8. Februar 2005 (2005-02-08) * das ganze Dokument * | 1-10 | |
| A | L. HEINEMANN: "Continuous glucose monitoring by means of the microdialysis technique: underlying fundamental aspects" DIABETES TECHNOLOGIES & THEURAPEUTICS, Bd. 5, Nr. 4, 2003, Seiten 545-561, XP008059493 * das ganze Dokument * | 1-10 | RECHERCHIERTE SACHGEBIETE (IPC) G01N |
| A | W.E. ELMQUIST, R.J. SAWCHUK: "Application of microdialysis in pharmacokinetic studies" PHARMACEUTICAL RESEARCH, Bd. 14, Nr. 3, 1997, Seiten 267-288, XP002366231 * das ganze Dokument * | 1-10 | |

Der vorliegende Recherchenbericht wurde für alle Patentansprüche erstellt

| Recherchenort | Abschlußdatum der Recherche | Prüfer |
|---|---|---|
| München | 7. Februar 2006 | Stussi, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

# EP 1 783 484 A1

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-02-2006

| Im Recherchenbericht angeführtes Patentdokument | | Datum der Veröffentlichung | Mitglied(er) der Patentfamilie | | Datum der Veröffentlichung |
|---|---|---|---|---|---|
| US 2004168934 | A1 | 02-09-2004 | WO | 03003911 A2 | 16-01-2003 |
| | | | AT | 412060 B | 27-09-2004 |
| | | | AT | 10572001 A | 15-02-2004 |
| | | | EP | 1404217 A2 | 07-04-2004 |
| WO 9716726 | A | 09-05-1997 | AU | 7327196 A | 22-05-1997 |
| | | | DE | 69621634 D1 | 11-07-2002 |
| | | | DE | 69621634 T2 | 19-09-2002 |
| | | | EP | 0858603 A1 | 19-08-1998 |
| US 6852500 | B1 | 08-02-2005 | AT | 268141 T | 15-06-2004 |
| | | | DE | 19935165 A1 | 01-02-2001 |
| | | | EP | 1072222 A2 | 31-01-2001 |
| | | | JP | 2001066313 A | 16-03-2001 |

EPO FORM P0461

Für nähere Einzelheiten zu diesem Anhang : siehe Amtsblatt des Europäischen Patentamts, Nr.12/82